# EUROPEAN PATENT SPECIFICATION

(11) **EP 0 503 507 B1**
(45) Date of publication and mention of the grant of the patent: **23.07.1997**
(21) Application number: 92103901.2
(22) Date of filing: 06.03.1992
(51) Int. Cl.: A61K 7/06, A61K 7/13

(54) **Dye composition for keratinous fibers**
Haarfärbemittel für keratinische Fasern
Composition tinctoriale pour fibres kératiniques

(30) Priority: 08.03.1991 JP 43782/91
(43) Date of publication of application: 16.09.1992
(73) Proprietor: Kao Corporation, Chuo-ku Tokyo (JP)
(72) Inventor: Yoshihara, Toru, Tokyo (JP); Misu, Daisuke, W-6102 Pfungstadt-Hahn (DE)
(74) Representative: Wächtershäuser, Günter, Prof. Dr.

(56) References cited:
- EP-A- 0 089 749
- EP-A- 0 137 178
- EP-A- 0 217 274
- EP-A- 0 257 807
- EP-A- 0 470 381
- US-A- 3 986 825

## Description

The present invention relates to a dye composition for keratinous fibers, and, in particular, to a dye composition providing a long-lasting excellent conditioning effect.

### Description of the Background Arts:

Most popular oxidative hair dyes consist essentially of an oxidative dyestuff and an oxidizing agent. Since they are used together with hydrogen peroxide under alkaline conditions, the dyeing operation involves risks of damaging hair and of giving primary irritation to the skin, producing red spots, small blisters, and the like. In order to eliminates these risks to scalps and hairs by oxidative dyeing, semipermanent hair dyes comprising a direct dye and imparting less adverse effects to scalps and hairs have been developed.

Problems in these semipermanent hair dyeing compositions are providing unfavorable feelings upon finish because of a comparatively large amount of solvents contained therein. Hairs treated with these dye compositions cannot be combed smoothly with finger; they are rather hard and less slippery.

Japanese Patent Laid-open (ko-kai) No. 50145/1974 discloses a semipermanent hair dye composition in which a quarternary amine compound and an N-oxyalkylated long chain fatty acid amide are used in combination, claiming that the composition improves the feel of hair and prevents hair from being entangled, thus ensuring its easy combing. A problem in this type of hair dye composition was that it tends to be removed so easily from the hair by shampooing that its effects do not last long.

British Patent No. 2173515 discloses a method of promoting a hair conditioning effect by the combined use of a direct dye, a cationized silicone active agent, and a hydroxylated silicone derivative. The composition, however, has a problem that the conditioning effect does not last over a sufficient period time because a hydroxylated silicone derivative is easily removed from the hair.

Japanese Patent Laid-open (ko-kai) No. 157713/1983 discloses a hair dye composition comprising 0.5-10% of water-soluble cationic polymer and 0.5-30% of water-soluble anionic surfactant. The Patent Application claims that the hair dye composition can withstand several times of shampooing and provides an excellent hair-set effect. However, the combination of the polymers and the surfactants cannot provide a sufficiently long-lasting conditioning effect.

EP-A-470381 discloses a composition comprising a nitro-dye, a cationic polymer (JR-400), softazoline, ethanol and 2-benzyloxyethanol.

EP-A-257807 discloses a composition comprising food blue or acid black, merquat 100, Miranol C2M and benzylalcohol.

EP-A-89749 discloses a composition comprising a direct dye, a polymer, an organic solvent and a betaine-type detergent.

EP-A-217274 discloses a composition comprising a direct dye, an ampholytic polymer, a betaine and an organic solvent.

US-A-3986825 discloses a composition comprising a direct dye (FD&C Blue No. 2), polydiallyldimethylammonium chloride, Miranol C2M and benzylalcohol.

There have been no dye composition for keratinous fibers using a direct dye which provides a good feel and a long-lasting excellent conditioning effect which is not lost by shampooing or the like. Development of such a composition has earnestly been desired.

In view of this situation the present inventors have undertaken extensive studies and found that a composition exhibiting a long-lasting excellent hair conditioning effect can be obtained by incorporating a betaine derivative in a direct dye composition together with a cationic polymer or an amphoteric polymer. This finding has led to the completion of the present invention.

### SUMMARY OF THE INVENTION

Accordingly, an object of the present invention is to provide a dye composition for keratinous fibers comprising:
(a) a direct dye selected from the group consisting of nitro dye, basic dye, disperse dye, and mixtures thereof,
(b) a polymer or copolymer of diallyl quaternary ammonium salt,
(c) a betaine-type surfactant selected from:
   (1) a carbobetaine-type surfactant represented by formula (I); 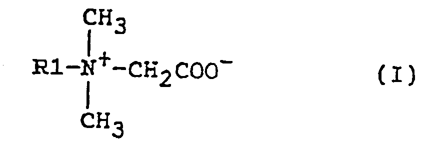 wherein R1 denotes a C10-24 straight or branched alkyl, or 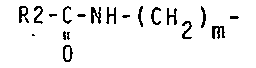 (wherein R2 is a C9-23 straight or branched alkyl, m denotes an integer of 1-5) or;
   (2) a sulfobetaine-type surfactant represented by formula (II): 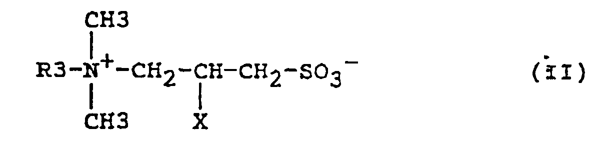 wherein R3 denotes a C10-24 straight or branched alkyl or, 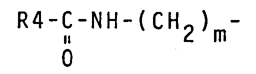 (wherein R4 denotes a C9-23 straight or branched alkyl, m denotes an integer of 1-5), X denotes hydrogen atom or hydroxyl, and,
(d) an organic solvent.

Other objects, features and advantages of the invention will hereinafter become more readily apparent from the following description.

### DETAILED DESCRIPTION OF THE INVENTION AND PREFERRED EMBODIMENTS

Direct dyes which can be used in the present invention are any dyes which can directly dye keratinous fibers in a dying bath without using mordants. They are nitro dyes, basic dyes, disperse dyes, and mixtures thereof. Specific examples of nitro dyes are 3-amino-4-hydroxynitrobenzene, 2-amino-5-hydroxynitrobenzene, 2-amino-3-hydroxynitrobenzene, 2-amino-5-N,N-bis-β-hydroxyethylaminonitrobenzene, 2-amino-4-chloro-5-N-β-hydroxyethylaminonitrobenzene, 2-amino-4-methyl-5-N-β-hydroxyethylaminonitrobenzene, 3,4-bis-(N-β-hydroxyethylamino)nitrobenzene, 2-amino-4-methyl-5-N-β,γ-dihydroxypropylaminonitrobenzene, 2-amino-4-methyl-5-β-aminoethylaminonitrobenzene, 2-amino-4-hydroxynitrobenzene. Furthermore, given as particularly preferable dyes are 3,4-diaminonitrobenzene, 2,5-diaminonitrobenzene, 2-amino-5-β-N-hydroxyethylaminonitrobenzene, 2-N-β-hydroxyethylamino-5-N,N-bis-β-hydroxyethylaminonitrobenzene, 2-N-methylamino-5-N,N-bis-(β-hydroxyethyl)aminonitrobenzene, 2-N-methylamino-5-N-methyl-N-β-hydroxyethylaminonitrobenzene, 2-N-β-hydroxyethylamino-5-hydroxynitrobenzene, 3-methoxy-4-N-β-hydroxyethylaminonitrobenzene, (nitro-4-methylamino-3)phenoxyethanol, 2-N-β-hydroxyethylamino-5-aminonitrobenzene, 2-N-β-hydroxyethylaminonitrobenzene, 3-amino-4-N-β-hydroxyethylaminonitrobenzene, 3-β-hydroxyethyloxy-4-N-β-hydroxyethylaminonitrobenzene, 2-amino-5-N-methylaminonitrobenzene, 2-amino-3-methylnitrobenzene, 2-N-β-hydroxyethylamino-5-β,γ-dihydroxypropyloxynitrobenzene, 3-hydroxy-4-aminonitrobenzene, 3-hydroxy-4-N-β-hydroxyethylaminonitrobenzene, 2,5-N,N'-β-hydroxyethylaminonitrobenzene, 2-N-methylamino-4-o-β,γ-dihydroxypropyloxynitrobenzene, 2-N-β-aminoethylamino-5-N,N-bis-(β-hydroxyethyl)aminonitrobenzene, 2-N-β-aminoethylamino-4-methoxynitrobenzene, 2-N-β-aminoethylamino-5-β-hydroxyethyloxynitrobenzene, 1-amino-4-methylaminoanthraquinone, 1,4-diaminoanthraquinone like.

Examples of basic dyes are Siena Brown, Mahogany, Madder Red, Steel Blue, Straw Yellow (all trademarks, products of William Co.), and the like. Given as examples of disperse dyes are Disperse Black 9, Disperse Blue 1, Disperse Violet 1, Disperse Violet 4 (all described in Cosmetic Ingredient Dictionary, U.S.A.), and the like. These dyes are incorporated in the composition of the present invention in an amount of 0.05-3% by weight ("% by weight" is hereinafter simply referred to as "%"), preferably 0.1-1%, although a specific amount is determined within this range depending upon the color of the dye.

Cationic polymers which is component (b) in the composition of the present invention may be copolymers of diallyl quaternary ammonium salt and acrylamide or polymers of diallyl quaternary ammonium salt.

Desirable polymers of cationized diallyl quaternary ammonium salt and copolymers of cationized diallyl quaternary ammonium salt and acryl amide for use in the present invention are those represented the following formulae (4) and (5). 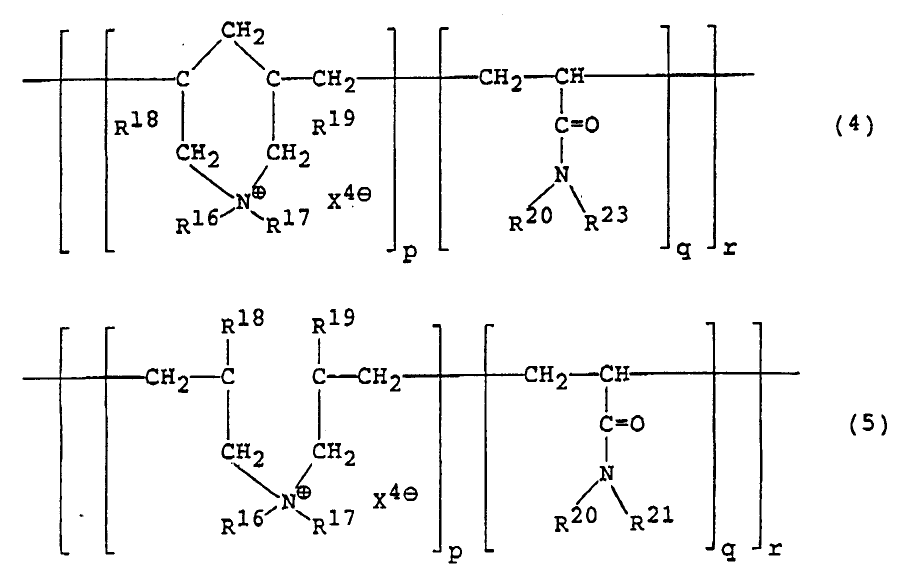 wherein R¹⁶ and R¹⁷ may be the same or different and each independently represents a hydrogen, an alkyl group with 1-18 carbon atoms, a phenyl group, an aryl group, a hydroxyalkyl group, an amidoalkyl group, a cyanoalkyl group, an alkoxyalkyl group, or a carboalkoxyalkyl group, R¹⁸, R¹⁹, R²⁰, and R²¹ may be the same or different and each independently represents a hydrogen, a lower alkyl group with 1-3 carbon atoms, or a phenyl group, X⁴ represents an anion; e.g., chlorine, bromine, iodine, sulfate, sulfonate, methylsulfate, phosphate, nitrate, or the like, p is an integer of 1-50, q is an integer of 0-50, and r is an integer of 150-8,000.

The molecular weights of the polymers of diallyl quaternary ammonium salt and the copolymers of diallyl quaternary ammonium salt and acryl amide may be in the range of 30,000-2,000,000, with the range 100,000-1,000,000 being preferable.

A preferable amount of cationic nitrogen of the cationic polymer contained in the above vinyl polymers is 0.004-0.2%, and preferably 0.01-0.15%, of the vinyl polymer. If the amount of the cationic nitrogen is smaller than 0.004%, the effects of the present invention is not sufficiently exhibited. The amount exceeding 0.2% may cause the vinyl polymer colored and is uneconomical, even though a good performance can be provided by such an excess amount.

The betaine-type surfactants; component (c) of the composition of the present invention; are compounds of the following formulas (9) or (10) 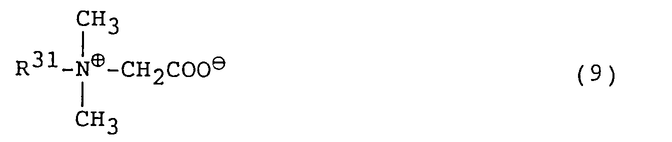 wherein R³¹ is a linear or branched alkyl group having 10-24 carbon atoms, or a group represented by formula, 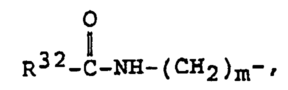 wherein R³² is a linear or branched alkyl group having 9-23 carbon atoms and m is an integer of 1-5. 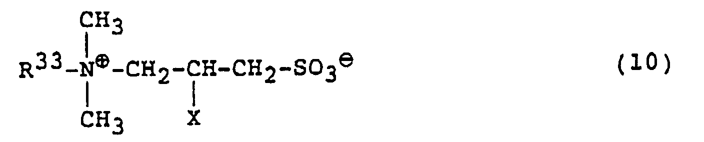 wherein R³³ is a linear or branched alkyl group having 10-24 carbon atoms, or a group represented by formula, 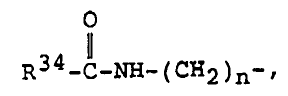 wherein R³⁴ is a linear or branched alkyl group having 9-23 carbon atoms and n is an integer of 1-5; and X is a hydrogen or a hydroxy group.

Specific examples of the betaine-type surfactants are cocamidopropylbetaine, oleamidopropylbetaine, lauramidopropylbetaine, cocobetaine, oleylbetaine, laurylbetaine, cetylbetaine, cocamidopropylhydroxy sultain, lauryl saltain, and the like. Of these especially preferable are cocamidopropylbetaine, lauramidopropyl-betaine, and the like. Here, "coco" means fatty acid mixtures of which the major components are C₁₂ fatty acids derived from coconut oil.

The amount of components (c) to be formulated in the composition of the present invention is preferably 0.1-0.5%. An amount outside this range does not give the intended long-lasting conditioning effect.

There are no limitations as to organic solvents (d) used in the composition of the present invention, so long as the solvent can dissolve components (b), cationic polymer, etc., and betaine derivatives (c). Examples of preferable organic solvents include ethanol, iso-propanol, n-propanol, n-butanol, iso-butanol, ethylene glycol, propylene glycol, 1,3-butanediol, benzyl alcohol, cinnamyl alcohol, phenetyl alcohol, p-anisyl alcohol, p-methylbenzyl alcohol, phenoxy ethanol, 2-benzyloxy ethanol, methyl carbitol, ethyl carbitol, propyl carbitol, butyl carbitol, triethylene glycol monoethyl ether, triethylene glycol monobutyl ether, glycerin, N-methylpyrrolidone, N-octylpyrrolidone, N-laurylpyrrolidone, and the like. They may be used alone or two or more of them may be used together.

Complexes of components (b) and (c) dissolved in the organic solvent, are diluted when hair is rinsed, and deposit on the hair, thus exhibiting excellent conditioning effects. In order to promote the deposition of complexes on the hair, the solvent is desirably used as a mixture with water. A preferable proportion of the solvent and water is in the range of 20:80-50:50, although a specific proportion depends on the solubility of the water-soluble complex in the solvent. If the proportion of water is too high, the complex tends to deposit in the aqueous system, thus impairing the conditioning effect. If the amount of water is too small or if no water is added at all, the amount of the complex deposited on the hair is insufficient, resulting in a reduced conditioning effect.

Other components which are commonly used in cosmetic compositions, such as thickners (e.g., hydroxyethylcellulose), oil components (e.g., silicones), perfumes, preservatives, UV absorbers, antiseptics, and the like, may optionally be incorporated to the extent that they do not impair the effects intended in the present invention.

Other features of the invention will become apparent in the course of the following description of the exemplary embodiments which are given for illustration of the invention and which are not intended to be limiting thereof.

### EXAMPLES

In the examples below in order to evaluate compositions of the present invention and comparative compositions 2.5 g of each composition was applied to a tress of blond hair weighing about 5 g, left for 30 minutes at 30°C, washed with a stream of water, shampooed and rinsed, and dried. The tresses were evaluated 5 expert panelists on the following items.

### (1) Smoothness

- AAA:: Much smoother than untreated hair.
- BBB:: Smoother than untreated hair.
- CCC:: The smoothness is equivalent to untreated hair.
- DDD:: Less smooth than untreated hair.

### (2) Feel of combing

- AAA:: Can be combed much better than untreated hair.
- BBB:: Can be combed better than untreated hair.
- CCC:: Can be combed as well as untreated hair.
- DDD:: Combed worse than untreated hair.

### (3) Feel of finger combing

- AAA:: Can be combed with finger much better than untreated hair.
- BBB:: Can be combed with finger better than untreated hair.
- CCC:: Can be combed with finger as well as untreated hair.
- DDD:: Combed with finger worse than untreated hair.

### (4) Feel of touch

- AAA:: The feel is much better than untreated hair.
- BBB:: The feel is better than untreated hair.
- CCC:: The feel is equivalent to untreated hair.
- DDD:: The feel is worse than untreated hair.

Each of the dyed hair tresses was divided into two portions; one of the portions was shampooed, rinsed, and dried 5 times for comparison with another portion which has not been shampooed on the following items.

### (1) Smoothness

- AAA:: The smoothness is equivalent to non-shampooed hair.
- BBB:: Slightly less smooth than non-shampooed hair.
- CCC:: Less smooth than non-shampooed hair.
- DDD:: Considerably less smooth than non-shampooed hair.

### (2) Feel of combing

- AAA:: Can be combed as well as non-shampooed hair.
- BBB:: Combed slightly worse than non-shampooed hair.
- CCC:: Combed worse than non-shampooed hair.
- DDD:: Combed considerably worse than non-shampooed hair.

### (3) Feel of fFinger combing

- AAA:: Can be combed with finger as well as non-shampooed hair.
- BBB:: Combed with finger slightly worse than non-shampooed hair.
- CCC:: Combed with finger worse than non-shampooed hair.
- DDD:: Combed with finger considerably worse than non-shampooed hair.

### (4) Feel of touch

- AAA:: The feel is equivalent to non-shampooed hair.
- BBB:: slightly drier feel than non-shampooed hair.
- CCC:: Drier feel than non-shampooed hair.
- DDD:: Considerably drier feel than non-shampooed hair.

### Example 1

A dye (Steel Blue, 2-amino-5-β-N-hydroxyethylamino nitrobenzene) and Merquat 100 (Merck Co.) were dissolved in water. To the solution was added hydroxyethylcellulose suspended in a small amount of water, and the mixture was heated to about 60°C to increase its viscosity. After cooling to room temperature, ethanol, ethyl carbitol, and Softazoline LPB (a product of Kawaken Fine Chemical Co.) were added. The mixture was stirred to homogenize to obtain a gel-like dye composition for keratinous fibers (Invention Product No. 1).

Invention Product No. 2 and Comparative product Nos. 1-3 were prepared in the same manner as Invention Product No. 1.

The composition and the results of evaluation were shown in Tables 1-1 and 1-2, respectively. The results in Table 1-2 show a superior dyeing ability and a long-lasting excellent conditioning effect of Invention Products over Comparative Products.

**TABLE 1-1**

| (Composition) | | | | | |
|---|---|---|---|---|---|
| | Comparative product | | | Invention Product | |
| | 1 | 2 | 3 | 1 | 2 |
| Ethanol | 20.0% | 20.0% | 20.0% | 20.0% | 20.0% |
| Ethyl carbitol | 10.0 | 10.0 | 10.0 | 10.0 | 10.0 |
| Hydroxethylcellulose | 1.6 | 1.6 | 1.6 | 1.6 | 1.6 |
| Merquat 100 (act. 40%) *1 | 0.3 | 0.3 | - | 0.3 | - |
| Polymer JR-400 *2 | - | - | 0.3 | - | - |
| Yukafoamer-AM-75 (act. 30%) *3 | - | - | - | - | - |
| Merquat 550 (act. 8%) *4 | - | - | - | - | 0.3 |
| Miranol C2MSP (act. 71%) *5 | - | 0.3 | 0.3 | - | - |
| Softazoline LPB (act. 31%) *6 | - | - | - | 0.3 | 0.3 |
| Steel Blue *7 | 0.1 | 0.1 | 0.1 | 0.1 | 0.1 |
| Nitrobenzene | 0.3 | 0.3 | 0.3 | 0.3 | 0.3 |
| Water | ―Balance― | | | | |

| | | | | | |
|---|---|---|---|---|---|
| *1 Homopolymer of dimethyldiallylammonium chloride manufactured by Merck Co. | | | | | |
| *2 Cationized cellulose manufactured by Union Carbide Corp. | | | | | |
| *3 Copolymer of N-methacryloylethyl-N,N-dimethylammonium-α-N-methyl carboxybetaine and butyl acrylate (act. 30%) manufactured by Mitsubishi Petrochemical Co., Ltd. | | | | | |
| *4 Copolymer of dimethyldiallylammonium chloride and acryl amide manufactured by Merck Co. | | | | | |
| *5 Cocoanphocrboxypropionate manufactured by Miranol Co. | | | | | |
| *6 N-lauramidepropylbetaine manufactured by Kawaken Fine Chemical Co., Ltd. | | | | | |

**TABLE 1-2**

| (Evaluation Results) | | | | | |
|---|---|---|---|---|---|
| | Comparative product | | | Invention Product | |
| | 1 | 2 | 3 | 1 | 2 |
| Immediately after dyeing | | | | | |
| Smoothness | CCC | BBB | BBB | AAA | AAA |
| Feel of combing | CCC | BBB | BBB | AAA | AAA |
| Feel of finger combing | CCC | BBB | BBB | AAA | AAA |
| Feel of touch | CCC | CCC | CCC | AAA | BBB |

| After 5 time shampooing | | | | | |
|---|---|---|---|---|---|
| Smoothness | DDD | CCC | CCC | AAA | AAA |
| Feel of combing | DDD | CCC | CCC | AAA | AAA |
| Feel of finger combing | DDD | CCC | CCC | AAA | AAA |
| Feel of touch | DDD | DDD | DDD | AAA | BBB |

### Example 2

A dye composition for keratinous fibers was prepared from the following components in the same manner as the method for preparation of Invention Product No. 1.

| | |
|---|---|
| Ethanol | 20.0% |
| Benzyl alcohol | 5.0 |
| Hydroxyethylcellulose | 1.6 |
| Merquat 100 *1 | 0.3 |
| Miratain CDMB *2 | 0.3 |
| 2-amino-5-β-N-hydroxyethyl amino nitrobenzene | 0.4 |
| 1-amino-2-β-N-hydroxyethyl amino nitrobenzene | 0.1 |
| Perfume | 0.2 |
| Water | Balance |

| | |
|---|---|
| *1 Homopolymer of dimethyldiallylammonium chloride (act. 40%) manufactured by Merck Co. | |
| *2 Cocobetaine (act. 37%) manufactured by Miranol Co. | |

5 g of the above composition was applied to a tress of blond hair weighing about 10 g, left for 30 minutes at 30°C, washed with tap water, shampooed and rinsed, and dried. The hair was dyed into a bright chestnut brown color, very smooth, and could be combed very well with finger. These effects were well kept even after 5 time shampooing.

### Example 3

A dye composition for keratinous fibers was prepared from the following components in the same manner as the method for preparation of Invention Product No. 1.

| | |
|---|---|
| Ethanol | 20.0% |
| Benzyl alcohol | 5.0 |
| Hydroxyethylcellulose | 1.6 |
| Merquat 100 *1 | 0.3 |
| Miratain CB *2 | 0.3 |
| 2-amino-5-β-N-hydroxyethyl amino nitrobenzene | 0.3 |
| Perfume | 0.2 |
| Water | Balance |

| | |
|---|---|
| *1 Homopolymer of dimethyldiallylammonium chloride (act. 40%) manufactured by Merck Co. | |
| *2 Cocoamidepropylbetaine (act. 35%) manufactured by Miranol Co. | |

5 g of the above composition was applied to a tress of blond hair weighing about 10 g, left for 30 minutes at 30°C, washed with tap water, shampooed and rinsed, and dried. The hair was dyed into a blue color, very smooth, and could be combed very well with finger. These effects were well kept even after 5 time shampooing.

### Example 4

A dye composition for keratinous fibers was prepared from the following components in the same manner as the method for preparation of Invention Product No. 1.

| | |
|---|---|
| Ethanol | 10.0% |
| 1,3-Butanediol | 10.0 |
| Benzyl alcohol | 5.0 |
| Hydroxyethylcellulose | 1.6 |
| Merquat 100 *1 | 0.3 |
| Softazoline LPB *2 | 0.3 |
| Silicone derivative | 0.2 |
| Perfume | 0.2 |
| Madder Red | 0.3 |
| Water | Balance |

| | |
|---|---|
| *1 Homopolymer of dimethyldiallylammonium chloride (act. 40%) manufactured by Merck Co. | |
| *2 N-lauramidopropylbetaine (act. 31%) manufactured by Kawaken Fine Chemical Co., Ltd. | |

5 g of the above composition was applied to a tress of blond hair weighing about 10 g, left for 30 minutes at 30°C, washed with tap water, shampooed and rinsed, and dried. The hair was dyed into a red color, very smooth, and could be combed very well with finger. These effects were well kept even after 5 time shampooing.

As illustrated above, the present invention provides dye compositions for keratinous fibers exhibiting a superior dyeing ability and an excellent conditioning effect which is maintained for a long period of time with repeated shampooing.

Obviously, numerous modifications and variations of the present invention are possible in light of the above teachings. It is therefore to be understood that within the scope of the appended claims, the invention may be practiced otherwise than as specifically described herein.

## Claims

1. A dye composition for keratinous fibers comprising:
(a) a direct dye selected from the group consisting of nitro dye, basic dye, disperse dye, and mixtures thereof,
(b) a polymer or copolymer of diallyl quaternary ammonium salt,
(c) a betaine-type surfactant selected from:
(1) a carbobetaine-type surfactant represented by formula (I); 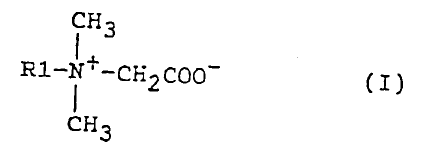 wherein R1 denotes a C10-24 straight or branched alkyl, or 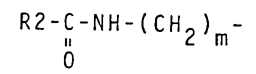 (wherein R2 is a C9-23 straight or branched alkyl, m denotes an integer of 1-5) or;
(2) a sulfobetaine-type surfactant represented by formula (II): 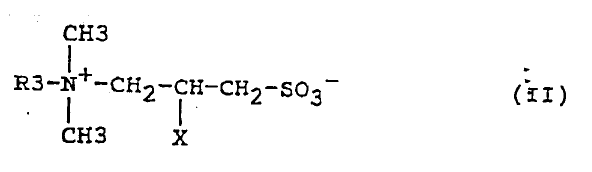 wherein R3 denotes a C10-24 straight or branched alkyl or, 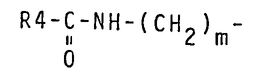 (wherein R4 denotes a C9-23 straight or branched alkyl, m denotes an integer of 1-5), X denotes hydrogen atom or hydroxyl, and,
(d) an organic solvent.

## Patentansprüche

1. Ein Haarfärbemittel für keratinische Fasern, enthaltend:
(a) einen aus der aus Nitrofarbstoffen, basischen Farbstoffen, Dispersionsfarbstoffen und deren Mischungen bestehenden Gruppe ausgewählten Direktfarbstoff,
(b) ein Polymer oder Copolymer eines quaternären Diallyl-Ammoniumsalzes,
(c) ein oberflächenaktives Mittel vom Betain-Typ, ausgewählt aus
(1) einem oberflächenaktiven Mittel vom Carbobetain-Typ der Formel (I); 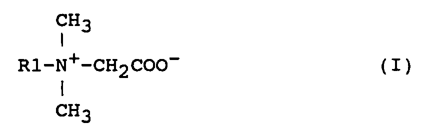 worin R1 eine geradkettige oder verzweigte C₁₀₋₂₄-Alkylgruppe oder 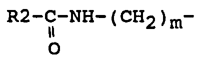 (worin R2 eine geradkettige oder verzweigte C₉₋₂₃-Alkylgruppe und m eine ganze Zahl von 1-5 bedeutet) oder;
(2) einem oberflächenaktiven Mittel vom Sulfobetain-Typ der Formel (II); 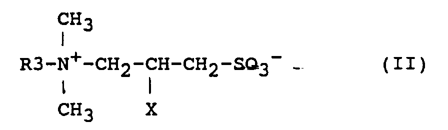 worin R3 eine geradkettige oder verzweigte C₁₀₋₂₄-Alkylgruppe oder 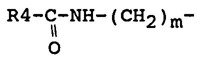 (worin R4 eine eine geradkettige oder verzweigte C₉₋₂₃-Alkylgruppe und m eine ganze Zahl von 1-5 bedeutet), X ein Wasserstoffatom oder Hydroxyl bedeutet und
(d) ein organisches Lösungsmittel.

## Revendications

1. Composition tinctoriale pour fibres kératiniques comprenant :
(a) un colorant direct choisi dans le groupe constitué des colorants nitro, des colorants basiques, des colorants dispersables et de leurs mélanges,
(b) un polymère ou un copolymère de sel de diallyl ammonium quaternaire,
(c) un dérivé tensio-actif de type bétaïne choisi parmi :
(1) un dérivé tensio-actif de type carbobétaïne représenté par la formule (I) : 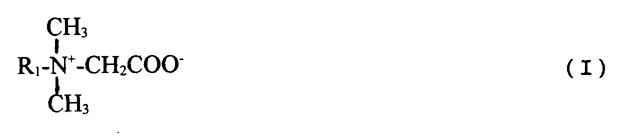 dans laquelle R₁ représente un groupe alkyle en C₁₀-C₂₄, linéaire ou ramifié ou 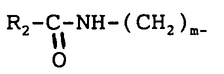 (où R₂ représente un groupe alkyle en C₉-C₂₃, linéaire ou ramifié, m désigne un nombre entier valant de 1 à 5) ou ;
(2) un dérivé tensio-actif de type sulfobétaïne représenté par la formule (II) : 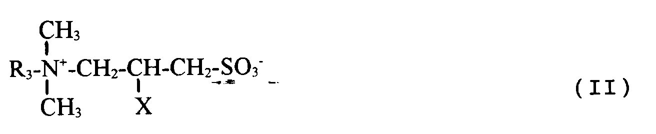 dans laquelle R₃ représente un groupe alkyle en C₁₀-C₂₄, linéaire ou ramifié ou 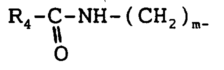 (où R₄ représente un groupe alkyle en C₉-C₂₃, linéaire ou ramifié, m désigne un nombre entier valant de 1 à 5), X représente un atome d'hydrogène ou un groupe hydroxyle, et
(d) un solvant organique.
